# EUROPÄISCHE PATENTSCHRIFT

(11) **EP 1 304 984 B1**
(45) Veröffentlichungstag und Bekanntmachung des Hinweises auf die Patenterteilung: **01.06.2005**
(21) Anmeldenummer: 01960534.4
(22) Anmeldetag: 23.07.2001
(51) Int. Cl.: A61F 2/46, A61F 2/36

(54) **CHIRURGISCHES ELEMENT**
SURGICAL ELEMENT
ELEMENT CHIRURGICAL

(30) Priorität: 29.07.2000 DE 10037345
(43) Veröffentlichungstag der Anmeldung: 02.05.2003
(73) Patentinhaber: Coripharm Medizinprodukte GmbH & Co. KG., 64807 Dieburg (DE)
(72) Erfinder: BUCHHOLZ, Jürgen, 42929 Wermelskirchen (DE)
(74) Vertreter: Helber, Friedrich G., Dipl.-Ing.
(86) Internationale Anmeldenummer: PCT/EP2001/008481
(87) Internationale Veröffentlichungsnummer: WO 2002/009627

(56) Entgegenhaltungen:
- EP-A- 0 434 604
- WO-A-94/07439
- WO-A-97/40785
- DE-A- 19 613 081
- FR-A- 2 720 268
- US-A- 4 274 163
- US-A- 4 653 487
- US-A- 5 702 446

## Beschreibung

Die Erfindung betrifft ein chirurgisches Applikations-, Halterungs-, Fixier- und/oder Befestigungsgelement mit einem zumindest bereichsweise langgestreckten Schaftabschnitt, wie z.B. Hüftgelenk-Endoprothesen mit einem im Markkanal des Femur eines Patienten zu fixierenden Schaft, Knochennägeln oder Knochenschrauben zur Frakturnagelung bzw. Lagefixierung von Knochenteilen nach Knochenoperationen etc., bei welchem der langgestreckte Schaftabschnitt eine an mindestens einem Ende zum Einbringen einer fließfähigen und nach dem Einbringen aushärtenden oder abbindenden Masse offen mündende, im wesentlichen in Längsrichtung des Schaftabschnitts verlaufende Durchgangsbohrung aufweist und wenigstens eine die Durchgangsbohrung mit der Außenseite des Schaftabschnitts verbindende Durchgangsöffnung vorgesehen ist.

Ein Prothesenteil für eine einzuzementierende Prothese gemäß dem Oberbegriff von Anspruch 1 ist aus Dokument DE-A-19 613 081 bekannt. Solche chirurgische Elemente mit Schaftabschnitten, die in entweder natürlich vorhandenen Knochenkanälen, wie dem Markkanal des Femur oder in vom Operateur mit chirurgischen Werkzeugen hergestellten Bohrungen festgelegt werden, um entweder Prothesenteile mit der für die spätere Belastung erforderlichen Festigkeit im jeweiligen Knochen zu befestigen bzw. um Knochenteile nach Knochenbrüchen in vorgegebener Ausrichtung zueinander zu fixieren, werden insbesondere in der orthopädischen Chirurgie in großem Umfang mit Erfolg eingesetzt. Problematisch ist die Anwendung solcher chirurgischen Hilfsmittel allerdings in den Fällen, in denen die Belastbarkeit des zur Aufnahme des Schaftabschnitts bestimmten Knochenteils verringert ist. Insbesondere bei älteren Patienten mit Osteoporose, aber auch jüngeren Patienten mit Trümmerbrüchen, kann die Anwendung von Knochennägeln - beispielsweise zur Versorgung von Schenkelhalsbrüchen oder die Festlegung des Schafts einer Hüftgelenk-Endoprothese im Markraumkanal -problematisch sein. Insbesondere bei Osteoporose-Patienten kann die Tragfähigkeit des Knochens - insbesondere in Spongiosa-Bereichen - so gemindert sein, dass eine Knochenschraube nicht mit hinreichender Belastbarkeit im Knochen festlegbar ist, so dass in solchen Fällen zusätzlich eine Ruhigstellung der betreffenden Gliedmaßen erforderlich ist, um Belastungen von der Befestigungsstelle so lange fernzuhalten, bis durch Bildung von neuem Knochengewebe die erforderliche Festigkeit erhalten wird. Hierbei besteht aber die Gefahr, dass ein in dieser Weise ruhiggestelltes Gelenk versteift und dass trotz Heilung des Knochenbruchs nach der Entfernung des Verbands keine oder keine volle Funktionsfähigkeit des betreffenden Gelenks mehr erreicht wird. Bei Trümmerbrüchen, auch von jüngeren Patienten, gelten ähnliche Überlegungen, wobei es in diesen Fällen auch darauf ankommt, nach dem Einrichten des Bruchs durch Einbringung eines Markraumnagels und Ausrichtung der Trümmerstücke zu verhindern, dass in die zwischen den Bruchstücken bestehenden Spalten und Hohlräume narbiges Weichteilgewebe einwächst. Hierzu ist wesentlich, dass sich in solchen Hohlräumen oder Spalten sammelndes Gewebswasser nach der Einrichtung des Bruchs verdrängt und mit einem Material gefüllt wird, welches verhindert, dass es zum Einwachsen von Weichteilgewebe kommt. Dieses in die Hohlräume bzw. Spalten eindringende Material kann entweder ein in bildsamem Zustand eingebrachter und dann aushärtender oder abbindender Knochenzement sein. In neuerer Zeit werden für solche Fälle aber vorzugsweise nach dem Erstarren oder Abbinden abbaubare, d.h. vom Körper nach und nach resorbierbare Materialien verwendet, wobei diese Materialien so zusammengesetzt sind, dass die Resorption zumindest angenähert in dem Maße erfolgt, in dem die genannten Hohlräume von neu gebildeten Knochenzellen durchwachsen werden. Insbesondere in bestimmter Weise zu verarbeitender Gips, der im Verarbeitungszustand in dem er steril mit Wasser angerührt wurde, pastös und fließfähig ist und deshalb mit Kanülen oder Spritzen ohne Eröffnung des umliegenden Weichteilbereichs direkt an die Defektstellen gebracht werden kann, wo er dann aushärten und die Defektstellen ausfüllt und eventuelle Knochenbruchstücke in ihrer Lage fixiert, wird hierfür verwendet. Dadurch wird der Bereich eines Trümmerbruchs zusätzlich zum eigentlichen metallischen Markraumnagel oder einer Knochenschraube stabilisiert und durch das Einbringen des aushärtenden Gipsmaterials werden die bestehenden Zwischenräume ausgefüllt und so das Gewebswasser verdrängt und dadurch das Einwachsen von Körpergewebe in die Zwischenräume verhindert. Der Gips wird dann relativ schnell vom Körper selbst resorbiert und durch neu entstehendes Knochengewebe ersetzt, wodurch dann sehr bald eine Eigenstabilität des Bereichs des Bruchs erreicht wird, der eine zusätzliche Fixierung von außen durch Gipsverbände bzw. - im Falle von Gelenkprothesen - eine Ruhigstellung des Gelenkbereichs zumindest erheblich verkürzt, so dass also auch Versteifungen von Gelenken in erheblich geringerem Maße zu befürchten sind, da bereits nach vergleichsweise kurzer Zeit nach der Operation schon wieder Gelenkbewegungen durchgeführt werden können. Für die Festlegung von Knochenschrauben in geschädigten spongiösen Knochenbereichen gilt sinngemäß Ähnliches. Hier müsste so verfahren werden, dass diese Bereiche unmittelbar bevor, während oder unmittelbar nach dem Einschrauben der Knochenschrauben mit der aushärtenden bzw. abbindenden resorbierbaren Masse ausgefüllt werden, damit hier nach dem Abbinden dieser Masse ein beanspruchbarer Knochenbereich entsteht, in welchem das Gewinde der Knochenschraube sich selbst hält. Anschließend wird das ausgehärtete Material dann wieder durch Resorption im Körper abgebaut und - im Idealfall in gleichem Maße - durch belastbares Knochengewebe ersetzt.

Aus den vorstehenden Überlegungen ergibt sich auch, dass der Einsatz von pastös, d.h. fließfähig verarbeitbaren und abbindenden oder aushärtenden Massen, die später im Körper resorbiert werden, zweckmäßig auch bei der Festlegung der Schäfte von Prothesen im Markraumkanal einzusetzen sind, und zwar anstelle des bisherigen Knochenzements, der ja nicht resorbiert wird, sondern lediglich den Zwischenraum zwischen dem Schaft der Prothese und der Markraumwandung ausfüllt.

Die Lösung der angesprochenen Probleme setzt voraus, dass während des Operationsvorgangs bei der Einbringung einer Prothese oder der Ausrichtung eines Bruchs im Verankerungsbereich von erforderlichen Prothesenschäften, Markraumnägeln oder Knochenschrauben das abbindende resorbierbare Material unter sterilen Bedingungen in den stabilisierenden Bereich einbringbar ist, ohne dass hierbei zusätzlich eine konventionelle operative Öffnung des die Bruchstelle umgebenden Weichteilgewebes des Patienten erforderlich ist.

Daraus stellt sich die der Erfindung zugrundeliegende Aufgabe dahingehend, dass die zur Stabilisierung von Brüchen bzw. Fixierung von Prothesen verwendeten chirurgischen Elemente so ausgebildet werden sollen, dass das resorbierbare abbindende Material mit den zur Befestigung oder Fixierung zu verwendenden chirurgischen Mitteln gezielt in den zu verfestigenden Bereich des Knochens unter Druck eingebracht werden kann, und zwar unabhängig davon, ob das chirurgische Hilfsmittel vorübergehend oder dauernd im Knochen verbleibt oder nur während des Einbringvorgangs des abbindenden Materials in den zu verfestigenden Bereich eingeführt und anschließend wieder entfernt wird.

Ausgehend von chirurgischen Elementen der eingangs erwähnten Art wird diese Aufgabe erfindungsgemäß dadurch gelöst, dass in der Durchgangsbohrung ein langgestrecktes hülsen- oder schlauchförmiges Verteilerelement zumindest partiell an der Wandung der Durchgangsbohrung anliegend und in der Durchgangsbohrung verschieblich und/oder verdrehbar angeordnet ist, welches mit wenigstens einer Durchgangsöffnung versehen ist, die durch Verschiebung und/oder Verdrehung des Verteilerelements mit jeweils einer Durchgangsöffnung im Schaftabschnitt ausrichtbar bzw. außer Flucht stellbar ist bzw. sind. Dadurch wird es möglich, die in ein Misch- und Applikationsgerät aus den Komponenten aufbereitete abbindende oder aushärtende Masse unter Druck ins Innere des Verteilerelements einzubringen und dann über eine oder mehrere Durchgangsöffnungen im Verteilerelement und mit diesen ausgerichtete Durchgangsöffnung im Schaftabschnitt in den benachbarten Knochenbereich austreten zu lassen. Durch Drehen oder Verschieben des Hülsenelements können dabei wahlweise Durchgangsöffnungen des Verstellelements und des Schaftabschnitts für den Durchtritt der noch pastösen Masse in Flucht gestellt bzw. zueinander versetzt werden, so dass es auf diese Weise möglich ist, die sich anschließend verfestigende Masse gezielt in die gewünschten Knochenbereiche zu applizieren.

Das hülsen- oder schlauchförmige Verteilerelement ist in bevorzugter Weiterbildung der Erfindung aus dem offenen Ende der Durchgangsbohrung herausgeführt und mündet an diesem Ende offen, so dass also die erwähnten Misch- und Applikationsgeräte ohne Schwierigkeiten am Verteilerelement angesetzt werden können.

Das in der Durchgangsbohrung angeordnete hülsen- oder schlauchförmige Verteilerelement kann - abhängig vom jeweiligen Anwendungsfall - an seinem dem offenen Ende der Durchgangsbohrung im Schaftabschnitt gegenüberliegenden Ende geschlossen sein oder offen münden.

Das bzw. die beiden offenen Enden des Verteilerelements sind zweckmäßig mit jeweils einem Adapter zum Anschluss an ein fließförmiges Material unter Druck zuführendes Applikationsgerät und/oder eine Unterdruckquelle versehen. Im Falle eines beidseitig offenen Verteilerelements kann dann der am äußeren Ende vorgesehene Adapter an das die fließfähige Masse unter Druck zuführende Applikationsgerät und der gegenüberliegende innere Adapter über geeignete Verbindungsleitungen oder Kanäle an die Unterdruckquelle angeschlossen werden. Über die Unterdruckquelle kann dann im Applikationsbereich befindliche Blut- oder Gewebeflüssigkeit abgesaugt werden, bevor die fließfähige Masse eingebracht wird.

Bei Verwendung eines nur an seinem äußeren Ende offenen Verteilerelements kann dieses durch eine Trennwand in zwei voneinander getrennte Kanäle unterteilt sein, wobei dann am äußeren offenen Ende des Verteilerelements an den beiden Kanälen jeweils ein eigener Adapter zum Anschluss an ein fließfähige Masse unter Druck zuführendes Applikationsgerät einerseits und eine Unterdruckquelle andererseits vorgesehen ist.

Dabei ist es zweckmäßig, wenn im Bereich der offenen Mündung der Durchgangsbohrung des chirurgischen Elements einerseits und im zugeordneten Endbereich des hülsen- oder schlauchförmigen Verteilerelements Markierungen vorgesehen sind, welche die relative Verschiebungs- und/oder Verdrehposition des Verteilerelements in der Durchgangsbohrung erkennen lassen. Anhand dieser äußeren Markierung ist es dann einfach möglich, die fluchtende Ausrichtung bzw. Versetzung von Durchgangsöffnungen im Verteilerelement und im Schaftabschnitt einzustellen, und so den Austrittsbereich der zu applizierenden abbindenden Masse aus dem Schaftabschnitt zu wählen.

Wenn der Schaftabschnitt mehrere in Schaftlängsrichtung zueinander versetzte Durchgangsöffnungen aufweist, kann die Ausgestaltung so getroffen sein, dass im langgestreckten hülsen- oder schlauchförmigen Verteilerelement eine der Anzahl und der relativen Positionierung der Durchgangsöffnung im Schaft entsprechende Anzahl und relativ zueinander positionierte Durchgangsöffnungen vorgesehen sind. Dann ist es möglich, die Durchgangsöffnungen im Schaftabschnitt und im Verteilerelement gleichzeitig in die fluchtende Stellung zu bringen bzw. außer Flucht zu stellen, so dass das so ausgebildete chirurgische Element die gleichzeitige Einbringung der fließfähigen Masse über die Länge des Schaftabschnitts verteilt ermöglicht.

Alternativ kann die Ausgestaltung auch so getroffen werden, dass das Verteilerelement eine der Anzahl der Durchgangsöffnungen im Schaftabschnitt entsprechende Anzahl von Durchgangsöffnungen aufweist, die jedoch in ihrer relativen Positionierung zueinander von der relativen Positionierung der Durchgangsbohrungen im Schaftabschnitt derart abweichen, dass eine fluchtende Ausrichtung der Durchgangsbohrungen im Verteilerelement und im Schaftabschnitt nur bezüglich jeweils einer oder eines Teils der Durchgangsöffnungen einstellbar ist. Die hierbei nicht in Flucht stehenden Durchgangsbohrungen im Schaftabschnitt und Verteilerelement können dann aber - erforderlichenfalls - durch eine entsprechende Verdrehung oder Verschiebung des Verteilerelements in der Durchgangsbohrung des Schaftabschnitts zur Flucht gebracht werden, wobei die vorher fluchtenden Durchgangsbohrungen außer Flucht gestellt werden. Auf diese Weise ist dann eine zeitlich aufeinanderfolgende Einbringung von aushärtender bzw. abbindender Masse in unterschiedlichen Knochen-Bereichen möglich.

Das Verteilerelement ist in bevorzugter Ausgestaltung der Erfindung zumindest in dem in bestimmungsgemäßer Montagestellung in der Durchgangsbohrung im Schaftabschnitt befindlichen Bereich als dünnwandiges Rohr aus Metall ausgebildet. Alternativ kommt auch eine Ausbildung des in der Durchgangsbohrung befindlichen Bereichs als dünnwandiger elastisch verbiegbarer Schlauch in Frage, der aufgrund seiner elastischen Verformbarkeit sich auch an Durchgangsbohrungen anpassen kann, welche keinen exakt geradlinigen Verlauf haben.

Ein solcher elastischer Schlauch ist dann zweckmäßig aus - ggf. durch eine Faser- oder Gewebeeinlage armiertem - Kunststoff hergestellt.

Die Erfindung ist in der folgenden Beschreibung eines Ausführungsbeispiels in Verbindung mit der Zeichnung näher erläutert, und zwar zeigt:

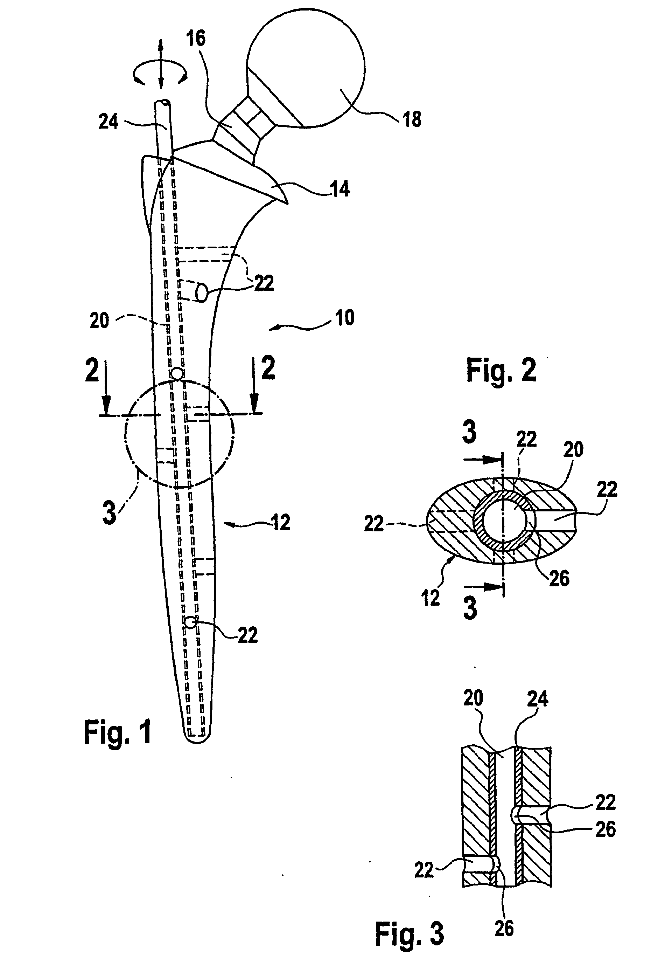

- Fig. 1: die Seitenansicht einer in der erfindungsgemäßen Weise ausgebildeten Hüftgelenk-Endoprothese;
- Fig. 2: eine Schnittansicht durch den Schaft der Prothese in der durch die Pfeile 2-2 in Fig. 1 veranschaulichten Ebene in vergrößertem Maßstab; und
- Fig. 3: eine Schnittansicht durch den innerhalb des in Fig. 1 strichpunktiert umschlossenen Bereichs 3 gelegenen Abschnitts des Schafts der Endoprothese in der in Fig. 2 durch die Pfeile 3-3 veranschaulichten Schnittebene.

Das in der Zeichnung dargestellte, in seiner Gesamtheit mit 10 bezeichnete Ausführungsbeispiel der Erfindung ist eine Hüftgelenk-Endoprothese, deren langgestreckter Schaft 12 nach Art des Zementschafts solcher Prothesen ausgebildet ist, welcher in bekannter Weise im Markkanal eines im proximalen Endbereich eines entsprechend chirurgisch vorbereiten Femur einsetzbar und mittels Knochenzement festlegbar ist. Bisher wird dabei so verfahren, dass vor dem Einführen des Schafts 12 in den entsprechend aufgeweiteten Markkanal nach Anbringung eines Markraumstopfens in geeigneter Tiefe Knochenzement in einer bestimmten Menge eingefüllt und dann der Schaft der Endoprothese bis in die bestimmungsgemäße Position, d.h. bis in Anlage des radial vergrößerten Kragens 14 an der am Ende des Femur ausgearbeiteten Anlagefläche eingeführt wird. Nach dem Abbinden des Knochenzements ist der Schaft 12 und somit auch der über dem Halsabschnitt 16 am Schaft angesetzte kugelförmige Gelenkkopf 18 der Prothese fest im Femur verankert.

In der Praxis kann aber nicht ganz ausgeschlossen werden, dass bei dieser Art der Festlegung des Schafts 12 im Markkanal im Knochenzement eingeschlossene Luftblasen verbleiben, welche eine Schwächung der Verbindung zwischen dem Schaft und dem Oberschenkelknochen darstellt. Bei der erfindungsgemäßen Endoprothese wird der Schaft 12 deshalb über seine gesamte Länge von einer Durchgangsbohrung 20 durchsetzt, von der in Längsrichtung versetzt nach unterschiedlichen Richtungen gerichtete Durchgangsöffnungen 22 zur Außenseite des Schafts 12 abzweigen. In die Durchgangsbohrung 20 ist ein langgestrecktes hohles zumindest in dem in der Durchgangsbohrung 20 liegenden Bereich röhrchenartiges Verteilerelement 24 eingesetzt, wobei die Passung zwischen der Außenseite des Verteilerelements 24 und der Wandung der Durchgangsbohrung 20 so gewählt, dass das Verteilerelement in der Durchgangsbohrung verdrehbar und/oder verschieblich ist. Im Verteilerelement 24 sind ebenfalls Durchgangsöffnungen 26 eingearbeitet, die - je nach relativer Dreh- bzw. Längspositionierung des Schafts 12 in der Durchgangsbohrung 20 mit zugeordneten Durchgangsöffnungen 22 des Schafts 12 ausgerichtet bzw. zu ihnen versetzt sind. Es ist nun klar, dass über das aus dem oberen Ende der Durchgangsbohrung 20 herausgeführten Ende des Verteilerelements 24 mittel eines geeigneten (bekannten) Applikationsgeräts eine im Einbringungszustand fließfähige, später abbindende oder aushärtende Masse, z.B. Knochenzement, unter Druck ins Verteilerelement und über in Flucht stehende Durchgangsöffnungen 26 und 22 dann in den Markkanal ausgepresst werden kann. Da die Einbringung durch entsprechende Ausrichtung bzw. außer Fluchtstellung der Durchgangsöffnungen 26, 22 so gesteuert werden kann, dass der Knochenzement zunächst an der Spitze des Schafts austritt und dann zum offenen Ende hin aufsteigend die Hohlräume zwischen der Außenwandung des Schafts und dem Markkanal ausfüllt, wobei eventuell in den Hohlräumen befindliche Luft in Richtung zum offenen Ende des Femur verdrängt wird, und außerdem durch entsprechende Steuerung der Drehlage bzw. der Längseinstellung des Verteilerelements 24 in der Durchgangsbohrung 20 aufeinander folgend, die in Schaftlängsrichtung nächstfolgenden Durchgangsöffnungen 22, 26 in Flucht gestellt werden können, sobald die Knochenzement-Füllung die jeweilige Bohrung erreicht hat, kann sichergestellt werden, dass der unter Druck eingebrachte Knochenzement den Zwischenraum zwischen dem Schaft und dem Markkanal des Oberschenkels vollständig und ohne Lufteinschlüsse ausfüllt. Ein sicherer und hoch belastbarer Sitz der Prothese im Markkanal ist also nach der Aushärtung des Knochenzements gewährleistet. Die nach dem Aushärten in den Durchgangsöffnungen 22 verbleibenden ausgehärteten Knochenzementansätze erbringen eine zusätzliche formschlüssige Halterung des Schafts im Markkanal über die stoffschlüssige Verbindung der Schaftaußenseite mit dem Femur durch den Knochenzement. Das Verteilerelement 24 kann in der Durchgangsbohrung 20 verbleiben, wobei dann nach dem Einbringen des Knochenzements der aus dem Schaft vortretende obere Endabschnitt des Verteilerelements abgetrennt wird. Alternativ kann das Verteilerelement 24 auch nach dem Einbringen des Knochenzements vor dessen vollständiger Aushärtung aus der Durchgangsbohrung 20 herausgezogen werden.

Zur Überwachung der relativen Lage der Durchgangsöffnung 22 und 26 im Schaft 12 bzw. dem Verteilerelement 24 können skalenartige Markierungen an der Außenfläche des Verteilerlements vorgesehen sein, die mit einer Markierung auf der Außenseite des oberen Schaftendes ausrichtbar sind und somit die relative Ausrichtung der Durchgangsöffnungen 26 und 22 zueinander erkennen lassen.

Das vorstehende in Verbindung mit der als Ausführungsbeispiel gewählten Hüftgelenk-Endoprothese 10 beschriebene Funktionsprinzip ist auch auf andere chirurgische Elemente wie Knochennägel, Knochenschrauben etc. übertragbar. Auch hier wird dann in dem im Knochen festzulegenden Schaftteil des betreffenden Elements eine Durchgangsbohrung mit abzweigenden Durchgangsöffnungen vorgesehen, wobei in der Durchgangsbohrung ein zugeordnetes hülsen- oder schlauchförmiges Verteilerelement mit Durchgangsöffnungen lageveränderlich eingesetzt ist. Durch relative Verdrehung bzw. Längsverschiebung des Verteilerelements im Schaftteil können die Durchgangsöffnungen im Schaftteil und Verteilerelement wahlweise ausgerichtet bzw. außer Flucht gestellt werden. Beim Einsatz solcher Knochennägel oder -schrauben zur Sanierung von Knochenfrakturen bei Patienten mit Osteoporose oder Trümmerbrüchen kann dann anstelle von Knochenzement ein fließfähiges nach dem Einbringen aushärtendes Material appliziert werden, welches im Körper allmählich resorbiert bzw. abgebaut wird, und zwar in dem Maße, in welchem die durch die Resorption entstehenden Hohlräume wieder von Knochenzellen durchwachsen werden.

Denkbar ist auch die Ausgestaltung von zum Einbringen solcher aushärtenden oder abbindenden, resorbierbarer Materialien bestimmter Kannülenelemente, über welche in bruchgefährdeten Knochenbereiche sozusagen vorsorglich und zeitlich vor chirurgischen Eingriffen ein aushärtendes oder abbindendes Material eingebracht wird, wodurch ein fester und belastbarer Sitz für später anschließend bei einer Bruchversorgung eingesetzte Knochennägel oder -schrauben üblicher Ausgestaltung erhalten wird. In Fällen, in denen sich in den durch Einbringen von fließfähigem, abbindendem oder aushärtendem Material zu stabilisierenden Knochenbereichen Blut-, Gewebeflüssigkeit oder o.dgl. befindet, kann das Kanülenelement auch so ausgebildet sein, dass vor dem Einbringen des fließfähigen, abbindenden oder aushärtenden Materials über das äußere offene Ende diese Blut- oder Gewebeflüssigkeit durch Anschluss des Kanüleninneren an eine Vakuumquelle abgesaugt werden kann.

## Patentansprüche

1. Chirurgisches Applikations-, Halterungs-, Fixier- und/oder Befestigungselement mit einem zumindest bereichsweise langgestreckten Schaftabschnitt, wie z.B. Hüftgelenk-Endoprothesen (10) mit einem im Markkanal des Femur eines Patienten zu fixierenden Schaft (12), Knochennägeln oder Knochenschrauben zur Frakturnagelung bzw. Lagefixierung von Knochenteilen nach Knochenoperationen etc., bei welchem der langgestreckte Schaftabschnitt (12) eine an mindestens einem Ende zum Einbringen einer fließfähigen und nach dem Einbringen aushärtenden oder abbindenden Masse offen mündende, im wesentlichen in Längsrichtung des Schaftabschnitts verlaufende Durchgangsbohrung (20) aufweist und wenigstens eine die Durchgangsbohrung (20) mit der Außenseite des Schaftabschnitts (12) verbindende Durchgangsöffnung (22) vorgesehen ist,
**dadurch gekennzeichnet,**
**dass** in der Durchgangsbohrung (20) ein langgestrecktes hülsen- oder schlauchförmiges Verteilerelement (24) zumindest partiell an der Wandung der Durchgangsbohrung (20) anliegend und in der Durchgangsbohrung verschieblich und/oder verdrehbar angeordnet ist, welches mit wenigstens einer Durchgangsöffnung (26) versehen ist, die durch Verschiebung und/oder Verdrehung des Verteilerelements (24) mit jeweils einer Durchgangsöffnung (22) im Schaftabschnitt (12) ausrichtbar bzw. außer Flucht stellbar ist bzw. sind.

2. Chirurgisches Element nach Anspruch 1, **dadurch gekennzeichnet, dass** das hülsen- oder schlauchförmige Verteilerelement (24) aus dem offenen Ende der Durchgangsbohrung (20) herausgeführt ist und an diesem Ende offen mündet.

3. Chirurgisches Element nach Anspruch 1 oder 2, **dadurch gekennzeichnet, dass** das in der Durchgangsbohrung angeordnete hülsen- oder schlauchförmige Verteilerelement (24) an seinem dem offenen Ende der Durchgangsbohrung (20) gegenüberliegende Ende geschlossen ist.

4. Chirurgisches Element nach Anspruch 1 oder 2, **dadurch gekennzeichnet, dass** das in der Durchgangsbohrung (20) angeordnete hülsen- oder schlauchförmige Verteilerelement (24) an seinem dem offenen Ende der Durchgangsbohrung (20) gegenüberliegende Ende offen mündet.

5. Chirurgisches Element nach Anspruch 3 oder 4, **dadurch gekennzeichnet, dass** das bzw. die beiden offenen Enden des hülsen- oder schlauchförmigen Verteilerelements (24) mit jeweils einem Adapter zum Anschluss an ein fließfähige Masse unter Druck zuführendes Applikationsgerät und/oder eine Unterdruckquelle versehen sind.

6. Chirurgisches Element nach Anspruch 3, **dadurch gekennzeichnet, dass** das hülsen- oder schlauchförmige Verteilerlement (24) in zwei voneinander getrennte Kanäle unterteilt ist, und dass am offenen Ende des Verteilerelements an jedem der beiden Kanäle jeweils ein Adapter zum Anschluss an ein fließfähige Masse zuführendes Applikationsberät einerseits und eine Unterdruckquelle andererseits vorgesehen ist.

7. Chirurgisches Element nach einem der Ansprüche 1 bis 6, **dadurch gekennzeichnet, dass** im Bereich der offenen Mündung der Durchgangsbohrung (20) des chirurgischen Elements (10) einerseits und im zugeordneten Endbereich des hülsen- oder schlauchförmigen Verteilerelements (24) Markierungen vorgesehen sind, welche die relative Verschiebungs- und/oder Verdrehungsposition des Verteilerelemens in der Durchgangsbohrung anzeigen.

8. Chirurgisches Element nach einem der Ansprüche 1 bis 7, bei welchem der Schaftabschnitt (12) mehrere, in Schaftlängsrichtung zueinander versetzte Durchgangsöffnungen (22) aufweist, **dadurch gekennzeichnet, dass** im langgestreckten hülsen- oder schlauchförmigen Verteilerelement (24) eine der Anzahl und der relativen Positionierung der Durchgangsöffnungen (22) im Schaft (12) entsprechende Anzahl und relativ zueinander positionierte Durchgangsöffnungen (26) vorgesehen sind.

9. Chirurgisches Element nach einem der Ansprüche 1 bis 7, bei dem der Schaftabschnitt mehrere in Schaftlängsrichtung zueinander versetzte Durchgangsbohrungen aufweist, **dadurch gekennzeichnet, dass** das Verteilerelement (24) eine der Anzahl der Durchgangsöffnungen (22) im Schaftabschnitt (12) entsprechende Anzahl von Durchgangsöffnungen (26) aufweist, die jedoch in ihrer relativen Positionierung zueinander von der relativen Positionierung der Durchgangöffnungen (22) im Schaftabschnitt (12) derart abweichen, dass eine fluchtende Ausrichtung der Durchgangsbohrungen (26; 22) im Verteilerelement (24) und im Schaftabschnitt (12) nur bezüglich jeweils einer oder eines Teils der Durchgangsöffnungen (26; 22) einstellbar ist.

10. Chirurgisches Element nach einem der Ansprüche 1 bis 9, **dadurch gekennzeichnet, dass** das Verteilerelement (24) zumindest in seinem in bestimmungsgemäßer Montagestellung in der Durchgangsbohrung (20) im Schaftabschnitt (12) befindlichen Bereich als dünnwandiges Rohr aus Metall ausgebildet ist.

11. Chirurgisches Element nach einem der Ansprüche 1 bis 9, **dadurch gekennzeichnet, dass** das Verteilerelement (24) zumindest in seinem in bestimmungsgemäßer Montagestellung in der Durchgangsbohrung (20) im Schaftabschnitt (12) befindlichen Bereich als dünnwandiger elastisch verbiegbarer Schlauch ausgebildet ist.

12. Chirurgisches Element nach Anspruch 11, **dadurch gekennzeichnet, dass** der als dünnwandiger elastischer Schlauch ausgebildete Abschnitt des Verteilerelements (24) aus Kunststoff hergestellt ist.

13. Chirurgisches Element nach Anspruch 12, **dadurch gekennzeichnet, dass** das Material des als Schlauch aus Kunststoff ausgebildeten Bereichs des Verteilerelements (24) durch Faser- oder Gewebeeinlagen armiert ist.

## Claims

1. Surgical application, holding, fixing and/or fastening element with a shank portion which is elongated at least in some regions, such as for example hip endoprostheses (10) with a shank (12) to be fixed in the medullary channel of a patient's femur, bone pins or bone screws for pinning fractures and/or fixing the position of bone parts after bone surgery etc., in which the elongated shank portion (12) has a through bore (20) which extends substantially in the longitudinal direction of the shank portion and opens on at least one end for the introduction of a flowable mass which hardens or sets after introduction, and at least one through opening (22) is provided which connects the through bore (20) to the outside of the shank portion (12), **characterised in that** there is arranged in the through bore (20) an elongated distributor element (24) in the form of a sleeve or a hose which at least partially rests on the wall of the through bore (20) and is disposed so as to displaceable and/or rotatable in the through bore, and which is provided with at least one through opening (26) which can be aligned or moved out of alignment by displacement and/or rotation of the distributor element (24) with respect to a respective through opening (22) in the shank portion (12).

2. Surgical element as claimed in Claim 1, **characterised in that** the distributor element (24) in the form of a sleeve or hose is led out of the open end of the through bore (20) and opens at this end.

3. Surgical element as claimed in Claim 1 or 2, **characterised in that** the distributor element (24) in the form of a sleeve or hose which is disposed in the through bore is closed at its end lying opposite the open end of the through bore (20).

4. Surgical element as claimed in Claim 1 or 2, **characterised in that** the distributor element (24) in the form of a sleeve or hose which is disposed in the through bore is open at its end lying opposite the open end of the through bore (20).

5. Surgical element as claimed in Claim 3 or 4, **characterised in that** the open end or the two open ends of the distributor element (24) in the form of a sleeve or hose is/are each provided with an adapter for connection to an application device which supplies a flowable mass under pressure and/or to a vacuum source.

6. Surgical element as claimed in Claim 3, **characterised in that** the elongated distributor element (24) in the form of a sleeve or hose is divided into two channels which are separated from one another, and that an adapter for connection to an application device supplying a flowable mass on the one hand and a vacuum source on the other hand is provided at the open end of the distributor element on each of the two channels.

7. Surgical element as claimed in any one of Claims 1 to 6, **characterised in that** in the region of the open mouth of the through bore (20) of the surgical element (10) on the one hand and in the associated end region of the distributor element (24) in the form of a sleeve or hose provided markings are provided which indicate the relative displaced and/or rotated position of the distributor element in the through bore.

8. Surgical element as claimed in any one of Claims 1 to 7, in which the shank portion (12) has a plurality of through openings (22) which are offset relative to one another in the longitudinal direction of the shank, **characterised in that** in the elongated distributor element (24) in the form of a sleeve or hose a plurality of through openings (26) are provided, the number and the relative position thereof corresponding to the number and the relative position of the through openings (22) in the shank (12).

9. Surgical element as claimed in any one of Claims I to 7, in which the shank portion has a plurality of through openings which are offset relative to one another in the longitudinal direction of the shank, **characterised in that** the distributor element (24) has a plurality of through openings (26), the number of which corresponds to the number of through openings (22) in the shank portion (12), but which deviate in their relative position with respect to one another from the relative position of the through openings (22) in the shank portion (12) in such a way that an alignment of the through bores (26; 22) in the distributor element (24) and in the shank portion (12) can be adjusted only with respect to one of the through openings (26; 22) or a part of the through openings (26; 22) in each case.

10. Surgical element as claimed in any one of Claims 1 to 9, **characterised in that** the distributor element (24) is constructed as a thin-walled metal tube at least in the region thereof which in the prescribed installation position is located in the through bore (20) in the shank portion (12).

11. Surgical element as claimed in any one of Claims 1 to 9, **characterised in that** the distributor element (24) is constructed as a thin-walled hose which can be resiliently bent at least in the region in which in the prescribed installation position it is located in the through bore (20) in the shank portion (12).

12. Surgical element as claimed in Claim 11, **characterised in that** the portion of the distributor element (24) which is constructed as a thin-walled hose which can be resiliently bent is made from plastics material.

13. Surgical element as claimed in Claim 12, **characterised in that** the material of the region of the distributor element (24) which is constructed as a plastics hose is reinforced with fibre or fabric inserts.

## Revendications

1. Elément chirurgical d'application, d'attache, de serrage et/ou de fixation avec un segment de tige allongé du moins par zones, tel que par exemple des endo-prothèses de hanche (10) avec une tige (12) à fixer dans le canal médullaire du fémur d'un patient, des clous ou des vis à os pour le clouage de fractures ou la fixation de la position de parties osseuses suite à une opération des os, etc., dans lequel le segment de tige (12) allongé présente un perçage traversant (20) s'étendant pour l'essentiel dans la direction longitudinale du segment de tige et débouchant à au moins une extrémité de façon ouverte pour introduire une masse coulante et durcissante ou liante après l'introduction, et au moins une ouverture de passage (22) est prévue reliant le perçage traversant (20) à la face extérieure du segment de tige (12),
**caractérisé en ce que** dans le perçage traversant (20) un élément de répartition (24) allongé en forme de douille ou de tuyau est disposé du moins partiellement appliqué contre la paroi du perçage traversant (20) et mobile en déplacement et/ou en rotation dans le perçage traversant et qui est muni d'au moins d'une ouverture de passage (26) qui peut être alignée ou désalignée respectivement avec une ouverture de passage (22) dans le segment de tige (12) par déplacement et/ou rotation de l'élément de répartition (24).

2. Elément chirurgical selon la revendication 1,
**caractérisé en ce que** l'élément de répartition (24) en forme de douille ou de tuyau saille de l'extrémité ouverte du perçage traversant (20) et débouche de façon ouverte au niveau de cette extrémité.

3. Elément chirurgical selon la revendication 1 ou 2,
**caractérisé en ce que** l'élément de répartition (24) en forme de douille ou de tuyau disposé dans le perçage traversant est fermé au niveau de son extrémité située en regard de l'extrémité ouverte du perçage traversant (20).

4. Elément chirurgical selon la revendication 1 ou 2,
**caractérisé en ce que** l'élément de répartition (24) en forme de douille ou de tuyau disposé dans le perçage traversant (20) débouche de façon ouverte au niveau de son extrémité située en regard de l'extrémité ouverte du perçage traversant (20).

5. Elément chirurgical selon la revendication 3 ou 4,
**caractérisé en ce que** la ou les deux extrémités ouvertes de l'élément de répartition (24) en forme de douille ou de tuyau sont munies respectivement d'un adaptateur pour le raccordement à un appareil d'application amenant une masse coulante sous pression et/ou à une source de dépression.

6. Elément chirurgical selon la revendication 3,
**caractérisé en ce que** l'élément de répartition (24) en forme de douille ou de tuyau est divisé en deux canaux séparés l'un de l'autre, et un adaptateur est prévu à l'extrémité ouverte de l'élément de répartition sur chacun des deux canaux pour le raccordement à un appareil d'application amenant une masse coulante d'une part et à une source de dépression d'autre part.

7. Elément chirurgical selon l'une quelconque des revendications 1 à 6,
**caractérisé en ce que** dans la zone de la bouche ouverte du perçage traversant (20) de l'élément chirurgical (10) d'une part et dans la zone d'extrémité associée de l'élément de répartition (24) en forme de douille ou de tuyau d'autre part sont prévus des repères qui indiquent la position relative de déplacement et/ou de rotation de l'élément de répartition dans le perçage traversant.

8. Elément chirurgical selon l'une quelconque des revendications 1 à 7, dans lequel le segment de tige (12) présente plusieurs ouvertures de passage (22) décalées les unes par rapport aux autres dans la direction longitudinale de la tige,
**caractérisé en ce qu'**un nombre d'ouvertures de passage (26) positionnées les unes par rapport aux autres est prévu dans l'élément de répartition (24) allongé en forme de douille ou de tuyau, correspondant au nombre et au positionnement relatif des ouvertures de passage (22) dans la tige (12).

9. Elément chirurgical selon l'une quelconque des revendications 1 à 7, dans lequel le segment de tige présente plusieurs perçages traversants décalés les uns par rapport aux autres dans la direction longitudinale de la tige,
**caractérisé en ce que** l'élément de répartition (24) présente un nombre d'ouvertures de passage (26) correspondant au nombre des ouvertures de passage (22) dans le segment de tige (12), mais dont le positionnement relatif est toutefois différent du positionnement relatif des ouvertures de passage (22) dans le segment de tige (12), ne permettant de régler un alignement des perçages traversants (26 ; 22) dans l'élément de répartition (24) et dans le segment de tige (12) que par rapport à l'une des ouvertures de passage (26 ; 22) ou à une partie de celles-ci.

10. Elément chirurgical selon l'une quelconque des revendications 1 à 9,
**caractérisé en ce que** l'élément de répartition (24), du moins dans sa zone située en position de montage déterminée dans le perçage traversant (20) dans le segment de tige (12), est configuré comme un tube métallique à paroi mince.

11. Elément chirurgical selon l'une quelconque des revendications 1 à 9,
**caractérisé en ce que** l'élément de répartition (24), du moins dans sa zone située en position de montage déterminée dans le perçage traversant (20) dans le segment de tige (12), est configuré comme un tuyau à paroi mince déformable élastiquement.

12. Elément chirurgical selon la revendication 11,
**caractérisé en ce que** le segment de l'élément de répartition (24) en forme de tuyau élastique à paroi mince est réalisé en matière plastique.

13. Elément chirurgical selon la revendication 12,
**caractérisé en ce que** le matériau de la zone de l'élément de répartition (24) en forme de tuyau en matière plastique est armé par des inserts en fibre ou en tissu.
